# EUROPÄISCHE PATENTSCHRIFT

(11) **EP 3 764 928 B1**
(45) Veröffentlichungstag und Bekanntmachung des Hinweises auf die Patenterteilung: **23.11.2022**
(21) Anmeldenummer: 19714098.1
(22) Anmeldetag: 06.03.2019
(51) Int. Cl.: A61B 17/221

(54) **THROMBEKTOMIEVORRICHTUNG**
THROMBECTOMY DEVICE
DISPOSITIF DE THROMBECTOMIE

(30) Priorität: 12.03.2018 DE 102018105671
(43) Veröffentlichungstag der Anmeldung: 20.01.2021
(73) Patentinhaber: Phenox GmbH, 44801 Bochum (DE)
(72) Erfinder: BHOGAL, Pervinder, London EC1V 2NR (GB); HENKES, Hans, 70192 Stuttgart (DE); MONSTADT, Hermann, 44797 Bochum (DE); HANNES, Ralf, 44137 Dortmund (DE)
(74) Vertreter: Schneiders & Behrendt Bochum
(86) Internationale Anmeldenummer: PCT/EP2019/055543
(87) Internationale Veröffentlichungsnummer: WO 2019/174988

(56) Entgegenhaltungen:
- EP-A1- 2 926 747
- WO-A2-2009/132859
- US-A1- 2010 087 850
- US-A1- 2011 184 456
- US-A1- 2014 046 359
- US-A1- 2014 121 672
- US-A1- 2015 374 483
- US-A1- 2016 192 956
- US-A1- 2017 189 041

## Beschreibung

Die Erfindung betrifft eine Vorrichtung zur Entfernung von Thromben aus Blutgefäßen, wobei die Vorrichtung in einem expandierten Zustand, in dem sie im Blutgefäß freigesetzt wird, und in einem komprimierten Zustand vorliegt, in dem sie durch einen Mikrokatheter in das Blutgefäß eingeführt und aus diesem entfernt wird, wobei die Vorrichtung über einen proximalen, einen mittleren und einen distalen Abschnitt verfügt, der mittlere Abschnitt eine im Wesentlichen zylindrische Struktur aufweist und der proximale Abschnitt an eine Einführhilfe gekoppelt ist.

Thromboembolische Erkrankungen wie Herzinfarkt, Lungenembolie, periphere Thrombose, Organembolien etc. werden typischerweise durch einen Thromboembolus (im Folgenden kurz: Thrombus), d. h. einen viskoelastischen Blutklumpen aus Blutplättchen, Fibrinogen, Gerinnungsfaktoren etc. ausgelöst, der sich in einem Blutgefäß festgesetzt hat und dieses ganz oder teilweise verschließt. Der Verschluss von Organarterien führt dabei zu einer Unterbrechung der Versorgung des abhängigen Gewebes mit Sauerstoff und Nährstoffen. Der Störung des Funktionsstoffwechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thromboembolische Verschlüsse kommen auch gehäuft in den Bein- und Beckenvenen vor. Das Krankheitsbild eines thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urokinase oder mit Antikoagulantien zu behandeln, was der Thrombolyse oder der Eindämmung des Thrombenwachstums dient. Da diese Behandlungsmethoden meist zeitintensiv sind, werden sie oftmals mit Methoden kombiniert, die der Zerkleinerung oder Entfernung des Thrombus bzw. Embolus dienen.

Neben offenen chirurgischen Eingriffen kommen im Stand der Technik zunehmend transluminale bzw. endovaskuläre kathetergeführte interventionelle Therapieformen zum Einsatz, da diese weniger invasiv sind. So ist es bekannt, den Thrombus mittels Unterdruck erzeugenden Saugkathetern oder mechanisch mit Kathetern, welche mit Fangkörben, Wendeln, Haken oder dergleichen versehen sind, aus dem Körper des Patienten zu entfernen, siehe US 6,245,089 B1; US 5,171,233 A1, Thomas E. Meier et al., Stroke 2002 (9) 2232.

Der Nachteil thrombolytischer Behandlungsmethoden liegt darin, dass sie nach Ablauf des Zeitfensters nur noch selten Erfolg haben. Auch die bekannten transluminalen Vorrichtungen können einen Thrombus häufig nicht vollständig entfernen, wobei auch die Gefahr besteht, dass sich der Thrombus oder Fragmente davon freisetzen und im Blutstrom zu kleinlumigeren Gefäßen gelangen, wo sie schwerer zu erreichen und zu behandeln sind.

Aus der WO 2012/156069 A1 ist eine Thrombektomievorrichtung mit einem Schlitz bekannt, der sich wendelförmig über die Mantelfläche der Vorrichtung erstreckt, wobei ein Spannbügel den Schlitz am proximalen Ende wellenförmig überspannt. Nach Einfangen des Thrombus wird die Vorrichtung in einen Mikrokatheter (Aspirationskatheter) zurückgezogen und zusammen mit dem Thrombus aus dem Blutgefäßsystem entfernt. Diese Thrombektomievorrichtung eignet sich besonders zur Entfernung von Thromben aus kleinlumigen oder stark gewundenen Gefäßen wie denen des Gehirns.

US 2014/121672 A1 offenbart ein medizinisches Instrument in Stentform mit distalem Netz zum Auffangen von Thromben.

US 2010/087850 A1 offenbart eine stentförmige medizinische Vorrichtung zur Entfernung von Thromben aus Blutgefäßen mit einem Führungsdraht und distaler Membran.

Die im Stand der Technik bekannten Thrombektomievorrichtungen haben sich grundsätzlich bewährt, allerdings kann das Problem auftreten, dass sich der Thrombus nach dem Einfangen mit Hilfe der Vorrichtung aufgrund des stetigen Blutflusses erneut von der Vorrichtung löst. Insbesondere besteht bei weniger festen Thromben die Gefahr der Ablösung von Teilen des Thrombus. Abgelöste Thrombusteile können in andere Bereiche des Blutgefäßsystems gelangen und dort unter Umständen einen (ischämischen) Schlaganfall hervorrufen.

Erfindungsgemäß stellt sich somit die Aufgabe, eine Vorrichtung zur Entfernung von Thromben zur Verfügung zu stellen, mit der sich der Thrombus einfangen und aus dem Blutgefäßsystem entfernen lässt, die darüber hinaus aber auch verhindert, dass sich der Thrombus oder Thrombusfragmente aus der Vorrichtung lösen.

Diese Aufgabe wird erfindungsgemäß gelöst durch eine Vorrichtung zur Entfernung von Thromben aus Blutgefäßen, wobei die Vorrichtung in einem expandierten Zustand, in dem sie im Blutgefäß freigesetzt wird, und in einem komprimierten Zustand vorliegt, in dem sie durch einen Mikrokatheter in das Blutgefäß eingeführt und aus diesem entfernt wird, wobei die Vorrichtung über einen proximalen, einen mittleren und einen distalen Abschnitt verfügt, der mittlere Abschnitt eine im Wesentlichen zylindrische Struktur aufweist und der proximale Abschnitt an eine Einführhilfe gekoppelt ist, wobei der distale Abschnitt eine im Wesentlichen zylindrische Grundstruktur aufweist und sich zwischen mittlerem und distalem Abschnitt eine Einschnürung befindet, wobei der distale Abschnitt zumindest teilweise über eine Abdeckung durch eine Membran verfügt.

Auch aus dem Stand der Technik sind zwar bereits Thrombektomievorrichtungen bekannt, die im distalen Bereich über eine Polymerhaut zum Einfangen von Thrombusfragmenten verfügen, bei diesen ist die Membran jedoch so aufgespannt, dass Thrombusfragmente innerhalb der Vorrichtung von der Polymerhaut aufgefangen werden. Da die Polymerhaut für das Blut quasi undurchlässig ist, ergeben sich entsprechend Probleme beim Rückzug der Vorrichtung in den Mikrokatheter zwecks endgültiger Entfernung des Thrombus. Die Erfindung beruht nun darauf, dass eventuell auftretende Thrombusfragmente außerhalb der eigentlichen Vorrichtung, nämlich im Bereich der Einschnürung aufgefangen werden, wobei dann die im distalen Abschnitt aufgespannte Membran mit ihrer Außenseite dafür sorgt, dass die Thrombusfragmente nicht in weiter distal gelegene Bereiche des Blutgefäßsystems gelangen können. Beim Rückzug der Vorrichtung in den Mikrokatheter werden die in der Einschnürung aufgefangenen Thrombusfragmente somit entlang der Gefäßinnenwandung zurückgezogen, bis sie schließlich bei Rückzug der Vorrichtung in den Mikrokatheter mit in diesen aufgenommen werden. Hierbei ist zu beachten, dass es sich bei dem Mikrokatheter zur Aufnahme der Vorrichtung nebst Thrombus in der Regel um sog. Aspirationskatheter handelt, bei denen zugleich eine gewisse Sogwirkung ausgeübt wird, die zusätzlich dafür sorgt, dass der Thrombus und eingefangene Thrombusfragmente in den Aspirationskatheter gelangen.

Die Membran weist bei der Erfindung eine Trichterform auf, wobei das dünne Ende des Trichters zur Einschnürung weist und das breite Ende in Richtung distal. Die Membran liegt über ihren Umfang an der Innenwandung des Gefäßes an und kann Thrombusfragmente oder -abrieb auffangen. Unter einer Einschnürung wird erfindungsgemäß verstanden, dass der Durchmesser der Vorrichtung im Bereich der Einschnürung deutlich kleiner ist als sowohl der Durchmesser des mittleren Abschnitts als auch des distalen Abschnitts. Bevorzugt liegt der Durchmesser im Bereich der Einschnürung bei ≤ 80 % des Durchmessers im distalen und mittleren Abschnitt, bevorzugt ≤ 70 %, weiter bevorzugt ≤ 60 %, insbesondere bei ca. 50 %.

Die Anwendung der Vorrichtung erfolgt in der Weise, dass sie mithilfe eines Mikrokatheters an die Stelle im Blutgefäßsystem gebracht wird, an der der Thrombus entfernt wird. Hier wird die Vorrichtung im Bereich des Thrombus freigesetzt, um diesen einzuschließen. In der Regel erfolgt die Freisetzung im Thrombus selbst oder distal des Thrombus. Die Freisetzung erfolgt durch Vorschub der Vorrichtung mittels der Einführhilfe in Richtung distal bzw. Rückzug des Mikrokatheters in Richtung proximal bei gleichzeitiger Fixierung der Vorrichtung mittels der Einführhilfe. Unter Freisetzung wird verstanden, dass die Vorrichtung aus dem Mikrokatheter gelangt und sich entsprechend aufweiten kann. Da die Vorrichtung nicht dafür vorgesehen ist, dauerhaft im Blutgefäß zu verbleiben, bleibt die Vorrichtung in der Regel weiter mit der Einführhilfe verbunden. Bei der Einführhilfe handelt es sich zumeist um einen Führungsdraht. Sobald die Vorrichtung freigesetzt ist, nimmt sie einen expandierten Zustand an, in dem sie sich an das Gefäßlumen anpasst und an die Gefäßinnenwandung anlegt. Beim expandierten Zustand handelt es sich in der Regel um eine der Vorrichtung zuvor aufgeprägte Sekundärstruktur. Dies gilt insbesondere, wenn die Vorrichtung ganz oder teilweise aus einem Formgedächtnismaterial, insbesondere einem Formgedächtnismetall gefertigt ist. Die Aufprägung einer Sekundärstruktur erfolgt typischerweise durch eine Wärmebehandlung.

Der Thrombus wird durch die Vorrichtung eingefangen, entweder schon bei der Expansion der Vorrichtung und/oder beim Zurückziehen. Sobald sich der Thrombus in der Vorrichtung befindet oder auch die Vorrichtung den Thrombus teilweise durchsetzt, erfolgt der Rückzug der Vorrichtung in Richtung Mikro(Aspirations)katheter. Der Rückzug erfolgt durch Bewegung der Einführhilfe in Richtung proximal. Da die Einführhilfe an den proximalen Abschnitt der Vorrichtung gekoppelt ist, bewegt sich dieser entsprechend ebenfalls in Richtung Katheter, bis die Vorrichtung nebst Thrombus schließlich vom Katheter insgesamt aufgenommen wird. An der Gefäßwand anhaftende Teile des Thrombus werden durch die Scherwirkung der Streben mitgenommen. Der Thrombus wird in den Katheter eingezogen und mit dem Katheter aus dem Körper entfernt.

Wie bereits erwähnt, lässt man hierbei in der Regel einen gewissen Sog in Richtung Katheter wirken, um zusätzlich sicherzustellen, dass möglicherweise abgelöste Thrombusfragmente mit eingezogen werden.

Die Begriffe "proximal" und "distal" sind so zu verstehen, dass beim Einbringen der Vorrichtung zum behandelnden Arzt weisende Teile als proximal, vom behandelnden Arzt weg weisende Teile als distal bezeichnet werden. Die Vorrichtung wird somit typischerweise durch einen Mikrokatheter in distaler Richtung vorgeschoben. Der Begriff "axial" bezieht sich auf die von proximal nach distal verlaufende Längsachse der Vorrichtung, der Begriff "radial" auf hierzu senkrechte Ebenen.

Vorzugsweise weist zumindest der mittlere Abschnitt der Vorrichtung eine Vielzahl von über seine Mantelfläche verteilten offenen Zellen auf. Mit anderen Worten handelt es sich um eine Gitter- oder Maschenstruktur, aufgebaut aus Streben, sodass die zylindrische Grundstruktur auf der Mantelfläche eine Vielzahl von Öffnungen oder Maschen aufweist. Die Größe der offenen Zellen ist so bemessen, dass auf der einen Seite ein eingefangener Thrombus nicht durch diese hindurch treten kann, sondern sicher von der Vorrichtung gehalten wird, auf der anderen Seite ist der Blutfluss gewährleistet. Darüber hinaus ist die Maschenstruktur so konzipiert, dass die Streben bei der Freisetzung im Bereich eines Thrombus in diesen eindringen oder diesen auch komplett durchdringen können, um ihn festzuhalten.

Neben dem mittleren Abschnitt können auch der proximale und/oder der distale Abschnitt eine entsprechende Struktur haben, die auf der Mantelfläche verteilte offene Zellen aufweist. Insofern unterscheidet sich unter Umständen der Aufbau von proximalem, mittlerem und distalem Abschnitt nicht nennenswert, abgesehen davon, dass der distale Abschnitt durch eine Einschnürung vom mittleren Abschnitt getrennt und zumindest teilweise von einer Membran bedeckt ist, während der proximale Abschnitt in der Regel in Richtung der Einführhilfe/des Führungsdrahts seinen Durchmesser verringernd ausläuft.

Der Ausdruck "offene Zelle" bezieht sich auf die Gitterstruktur, unabhängig von der Frage, ob die Zelle durch eine Membran von der Umgebung abgekoppelt ist, d. h. auch eine von einer Membran abgedeckte Zelle wird als offene Zelle bezeichnet. Unter Abdeckung durch eine Membran wird jedwede Abdeckung einer Struktur verstanden, unabhängig davon, ob die Membran außen oder innen auf die Gitterstruktur aufgebracht oder ob die Gitterstruktur in eine Membran eingebettet ist.

Die Gitterstruktur der Vorrichtung bzw. zumindest des mittleren Abschnitts kann eine geflochtene Struktur sein, d. h. aus einzelnen Drähten bzw. Drahtbündeln als Streben bestehen, die miteinander verflochten sind und an den Kreuzungspunkten der Drähte/Drahtbündel über- und untereinander her verlaufen. Bevorzugt ist aber eine geschnittene Struktur, bei der aus einem Rohr geeigneten Durchmessers mit Hilfe eines Lasers die Gitterstruktur herausgeschnitten wird. Das Material ist in der Regel ein Metall, kann aber auch ein Kunststoff sein. Es muss über eine hinreichende Elastizität verfügen, die eine Kontraktion auf den Durchmesser eines üblichen Katheters erlaubt und andererseits bei der Freisetzung aus dem Katheter die Expansion auf den gewünschten Durchmesser. Zusätzlich ist es sinnvoll, die Gitterstruktur einer Elektropolitur zu unterziehen, um sie glatter und abgerundeter und damit weniger traumatisch werden zu lassen. Darüber hinaus sinkt die Gefahr der Anhaftung von Keimen oder sonstigen Verunreinigungen. Die Streben oder Drähte können einen runden, ovalen, quadratischen, rechteckigen oder trapezförmigen Querschnitt aufweisen, wobei im Falle eines quadratischen, rechteckigen oder trapezförmigen Querschnitts eine Abrundung der Kanten von Vorteil ist. Möglich ist auch die Verwendung von flachen Stegen/Drähten in Form dünner Streifen, insbesondere Metallstreifen. Bei den rechteckigen und trapezförmigen Ausführungsformen ist es bevorzugt, wenn die schmale Seite des Querschnitts zur Gefäßwand weist, was ein leichteres Eindringen des Thrombus in die Maschenstruktur ermöglicht.

Als Materialien kommen neben Eisenlegierungen (Edelstahl, Federstahl) und Kobalt-Chrom-Legierungen insbesondere Formgedächtnislegierungen in Frage, etwa binäre Nickel-Titan-Legierungen (Nitinol) und ternäre Nickel-Titan-Chrom-Legierungen (Chrom-dotierte Legierungen). Insbesondere Nitinol ist für die Anwendung in selbstexpandierenden Strukturen im neurovaskulären Bereich bekannt.

Die erfindungsgemäße Vorrichtung kann zunächst als flächige Struktur hergestellt werden, die anschließend zu einer zylindrischen Struktur gerollt wird. Dabei kann die Vorrichtung einen Schlitz aufweisen, der sich in Längsrichtung der Vorrichtung über die Mantelfläche zumindest eines Teils der Vorrichtung erstreckt. Der Schlitz kann beispielsweise parallel zur Längsachse der Vorrichtung oder auch wendel- oder helixförmig über die Mantelfläche verlaufen. Im letzteren Fall kann der Schlitz dabei eine vollständige Wendel von 360° darstellen, aber auch eine nur partielle von beispielsweise etwa 180° oder 120°. Ein wendelförmiger Verlauf des Schlitzes hat den zusätzlichen Vorteil, dass die Kanten der Vorrichtung entlang des Schlitzes bei Zug tangential entlang des Umfangs der Gefäßwandung wandern. Dies verbessert die Scherwirkung. Gleichzeitig verbessert (vermindert) sich durch den wendelförmigen Verlauf die Biegesteifigkeit dergestalt, dass eine bessere Anpassung an kurvige Gefäße möglich ist. Dies erleichtert sowohl die Platzierung als auch die Extraktion von Thromben aus komplexen Gefäßstrukturen. Die Mantelfläche der Vorrichtung klafft im Bereich dieses Schlitzes auf, wobei die Breite des Schlitzes am Einsatzort jeweils auch vom Lumen des Gefäßes bestimmt wird, da sich die Vorrichtung nach der Freisetzung aus dem Katheter nur so weit entfalten kann, wie es das Gefäßlumen zulässt.

Der Schlitz kann sich über die gesamte Länge der Vorrichtung erstrecken, bevorzugt ist jedoch, dass der Schlitz nur über einen Teil der Länge der Vorrichtung verläuft. Beispielsweise kann sich im proximalen Abschnitt der Vorrichtung ein Bügel über den Schlitz erstrecken, wie dies in der WO 2012/156069 A1 beschrieben wird. Ein solcher Bügel erhöht die Radialkraft der selbstexpandierenden Struktur, dient aber auch dazu, die einander gegenüberliegenden Kanten der Vorrichtung entlang des Schlitzes in ihrer Position zueinander zu fixieren.

Die Zahl der offenen Zellen, die orthogonal zur Längsachse der Vorrichtung auf der Mantelfläche nebeneinander liegen, beträgt mindestens 2, vorzugsweise jedoch 3 bis 5. Mit nebeneinander liegenden Zellen sind diejenigen Zellen gemeint, die bei einer aufgeschnittenen und flach ausgebreiteten Struktur orthogonal zur Längsachse der Vorrichtung nebeneinander liegen würden. Die richtige Auswahl der offenen Zellen hängt u. a. von der Größe des Blutgefäßes ab, in dem die Vorrichtung eingesetzt werden soll. Desto größer der Durchmesser des Blutgefäßes, desto größer wird in der Regel auch die Zahl der nebeneinander liegenden Zellen sein, da die Vorrichtung im expandierten Zustand insgesamt eine größere Umfangsfläche abdecken muss. Darüber hinaus gilt grundsätzlich, dass eine geringere Zahl an offenen Zellen eine flexiblere Vorrichtung bewirkt, was vorteilhaft sein kann, wenn die Vorrichtung innerhalb von eng gewundenen Blutgefäßen bewegt werden muss. Auf der anderen Seite bedeutet eine geringere Zahl von Öffnungen bei identischer Mantelfläche zugleich, dass die Größe der Öffnungen zunimmt. Die Größe der Öffnungen sollte so gewählt werden, dass ein Austreten des Thrombus oder von Thrombusfragmenten durch die Öffnungen hindurch möglichst vermieden wird. Insgesamt hat sich bewährt, Vorrichtungen zu verwenden, bei der die Zahl der offenen Zellen, die orthogonal zur Längsachse der Vorrichtung entlang des Umfangs auf der Mantelfläche nebeneinanderliegen, 3 bis 5 beträgt.

Als sinnvoll hat sich herausgestellt, wenn die im distalen Abschnitt der Vorrichtung angebrachte Membran über zumindest eine Öffnung verfügt. Diese ist vorzugsweise zentral angeordnet. Wenn die Membran insgesamt eine Trichterform aufweist, befindet sich die Öffnung somit am dünnen Ende des Trichters in der Mitte. Die Öffnung sorgt dafür, dass auch bei Verwendung einer im Übrigen weitgehend flüssigkeitsundurchlässigen Membran ein Blutfluss durch die Vorrichtung hindurch gewährleistet wird und sich die Gefahr einer Ischämie somit reduziert. Darüber hinaus erleichtert die Öffnung auch das Zurückziehen der Vorrichtung in Richtung Katheter, weil ein Aufstauen des Blutes verhindert wird.

Das Erzeugen der Einschnürung zwischen mittlerem und distalem Abschnitt erfolgt vorzugsweise in Form einer Wärmebehandlung. Mit anderen Worten wird zunächst die Vorrichtung mit ihrer normalen zylindrischen Struktur erzeugt, bevor anschließend über eine Wärmebehandlung gezielt eine Einschnürung erzeugt wird, die für die beschriebene Ausführungsform der Erfindung wesentlich ist. Die Vorrichtung wird hierzu erwärmt, unter diesen Bedingungen lässt sich eine Vorrichtung gerade bei Verwendung von Formgedächtnismaterialien wie Nitinol besonders gut umformen, um eine Einschnürung zu erreichen.

Gemäß einer alternativen Ausführungsform, welche unabhängig von der zuvor beschriebenen Ausführungsform zu betrachten ist, existiert eine Vorrichtung zur Entfernung von Thromben aus Blutgefäßen, wobei die Vorrichtung in einem expandierten Zustand, in dem sie im Blutgefäß freigesetzt wird, und in einem komprimierten Zustand vorliegt, in dem sie durch einen Mikrokatheter in das Blutgefäß eingeführt und aus diesem entfernt wird, wobei die Vorrichtung über einen proximalen, einen mittleren und einen distalen Abschnitt verfügt, zumindest der mittlere und der distale Abschnitt eine im Wesentlichen zylindrische Struktur aus Streben aufweist, die eine Vielzahl von über die Mantelfläche der Vorrichtung verteilten offenen Zellen ausbilden, und der proximale Abschnitt an eine Einführhilfe gekoppelt ist, wobei die Streben am distalen Ende des distalen Abschnitts nach innen gebogen sind und der distale Abschnitt zumindest teilweise über eine Abdeckung durch eine Membran verfügt.

Gemäß dieser alternativen Ausführungsform werden sich von der Vorrichtung lösende Thromben bzw. vom Thrombus lösende Thrombusfragmente vom distalen Ende der Vorrichtung, die von einer Membran bedeckt ist, aufgefangen. Entscheidend ist dabei, dass die Streben am distalen Ende des distalen Abschnitts nach innen gebogen sind und das distale Ende insgesamt abgerundet ist. Das distale Ende bildet somit eine Wölbung aus, die über offene Zellen bzw. Maschen verfügt. Auf diese Weise wird das distale Ende deutlich atraumatischer und die Gefahr der Verletzung von Gefäßwandungen wird verringert. Darüber hinaus sorgt die Tatsache, dass die Streben am distalen Ende nach innen gebogen sind, dafür, dass die Membran sicher gehalten wird und an keiner Stelle eine übermäßige Dehnung erfährt, da eine solche die Gefahr der Ruptur der Membran erhöhen würde. Dadurch, dass die Streben nach innen, d. h. in Richtung der zentral durch die Vorrichtung verlaufenden Längsachse der Vorrichtung umgebogen sind, hat das distale Ende der Vorrichtung insgesamt eine abgerundete Form, die sich innerhalb des Blutgefässes gut bewegen lässt.

Da bei dieser zweiten Ausführungsform die Membran in der Regel keine Öffnung aufweist, sollte die Membran für Flüssigkeiten, hier insbesondere Blut, zumindest teilweise permeabel sein. Auf diese Weise wird sichergestellt, dass der Blutfluss durch das Blutgefäß insgesamt erhalten bleibt. Außerdem wird der Rückzug der Vorrichtung in den Katheter erleichtert, wenn Blut durch die Membran hindurch von der einen Seite der Membran auf die andere, distal gelegene Seite gelangen kann.

Die Durchlässigkeit der Membran kann nach Bedarf eingestellt werden, um einen mehr oder weniger großen Filtereffekt zu erzielen. Auf der einen Seite ist eine hohe Durchlässigkeit vorteilhaft für den Blutfluss, auf der anderen Seite ist eine geringe Durchlässigkeit vorteilhaft zum Auffangen auch kleiner Thrombusfragmente.

Wie bei der ersten Ausführungsform ist es auch bei der zweiten Ausführungsform sinnvoll, wenn die Zahl der offenen Zellen, die orthogonal zur Längsachse der Vorrichtung auf der Mantelfläche nebeneinander liegen, mindestens 2, vorzugsweise 3 bis 5 beträgt. Hinsichtlich der Kriterien, die bei der Auswahl der optimalen Zahl an offenen Zellen angelegt werden, gilt das oben zur ersten Ausführungsform gesagte.

Allgemein gilt, dass die Ausführungen, die im Zusammenhang mit der ersten Ausführungsform gemacht wurden, in gleicher Weise für die zweite Ausführungsform gelten, soweit sich nicht aus dem Aufbau der Vorrichtung oder dem Zusammenhang etwas anderes ergibt. Insbesondere gelten die Ausführungen zu Gitter-/Maschenstruktur, dem Aufbau aus einzelnen offenen Zellen, verwendeten Materialien oder dem Vorsehen eines Schlitzes durch die Vorrichtung sowie zur Handhabung der Vorrichtung auch für die zweite Ausführungsform.

Das Umbiegen der Streben am distalen Ende des distalen Abschnitts erfolgt vorzugsweise in Form einer Wärmebehandlung. Mit anderen Worten wird zunächst die Vorrichtung mit ihrer normalen zylindrischen Struktur erzeugt, bevor anschließend über eine Wärmebehandlung eine gezielte Beeinflussung des Verlaufs der Streben am distalen Ende vorgenommen wird. Die Vorrichtung wird hierzu erwärmt, unter diesen Bedingungen lässt sich eine Vorrichtung gerade bei Verwendung von Formgedächtnismaterialien wie Nitinol besonders gut umformen, um eine Abrundung des distalen Endes zu erreichen.

Für beide Ausführungsformen gilt, dass es sich bei der Einführhilfe bevorzugt um einen Führungsdraht handelt, wie er hinlänglich im Stand der Technik bekannt ist. Die Einführhilfe kann an einem oder mehreren Kopplungspunkten an den proximalen Abschnitt der Vorrichtung gekoppelt sein. Typischerweise laufen die Streben der Vorrichtung im proximalen Abschnitt in Richtung der Einführhilfe zusammen. Dabei ist es zweckmäßig, wenn der oder die Kopplungspunkte zur zentral verlaufenden Längsachse der Vorrichtung in Richtung Mantelfläche verschoben angeordnet sind, d. h. radial außen. Eine solche exzentrische Anordnung des Kopplungspunkts ist mit dem Vorteil verbunden, dass der Blutfluss möglichst wenig gestört wird. Im aus dem Mikrokatheter ausgebrachten Zustand kann der Kopplungspunkt somit an der Gefäßwandung liegen.

Sinnvollerweise verfügt die Vorrichtung über ein oder mehrere röntgendichte Markierungen, um dem behandelnden Arzt eine Visualisierung zu ermöglichen. Die röntgendichten Markierungen können z. B. aus Platin, Palladium, Platin-Iridium, Tantal, Gold, Wolfram oder anderen röntgendichten Metallen sein. Sie erlauben es dem behandelnden Arzt zu erkennen, ob die Vorrichtung relativ zum zu entfernenden Thrombus korrekt platziert ist, und ggf. Korrekturen vorzunehmen. Denkbar ist auch, zumindest einige Bereiche der Vorrichtung mit einer Beschichtung aus einem röntgendichten Material zu versehen, beispielsweise mit einer Goldbeschichtung. Diese kann z. B. eine Stärke von 1 bis 6 µm aufweisen. Die Beschichtung mit einen röntgendichten Material muss nicht die gesamte Vorrichtung umfassen. Auch beim Vorsehen einer röntgendichten Beschichtung kann es allerdings sinnvoll sein, zusätzlich einen oder mehrere röntgendichte Markierungen anzubringen. Eine weitere Möglichkeit besteht darin, einzelne Streben mit einer Helix oder einem Draht aus einem röntgendichten Material wie Platin zu ummanteln.

Insbesondere bei der zweiten Ausführungsform ist es sinnvoll, röntgendichte Markierungen im Bereich des proximalen Endes der Abdeckung durch eine Membran vorzusehen, d. h. dort, wo von proximal aus gesehen die Abdeckung durch eine Membran beginnt. Der behandelnde Arzt kann beim Zurückziehen der Vorrichtung in einen Aspirationskatheter aufgrund der röntgendichten Markierung erkennen, wann das proximale Ende der Abdeckung an den distalen Einlass des Aspirationskatheters gelangt. Es kann sinnvoll sein, die Vorrichtung nicht weiter in den Aspirationskatheter zurückzuziehen, um ein "Herausquetschen" von eingefangenen Thrombusfragmenten zu vermeiden, und stattdessen den Aspirationskatheter mit der nicht vollständig eingezogenen Vorrichtung nach proximal zurückzubewegen und aus dem Blutgefäßsystem zu entfernen.

Dort, wo die Vorrichtung von einer Membran bedeckt ist, kann diese an der Gitterstruktur innen oder außen angebracht sein. Vorzugsweise ist die Gitterstruktur jedoch in die Membran eingebettet. Dies kann dadurch erreicht werden, dass zunächst eine Gitterstruktur vorgelegt wird, die anschließend so mit Fasern umsponnen bzw. umflochten wird, dass sich eine Membran mit eingebetteter Gitterstruktur ergibt. Entsprechende Verfahren sind aus dem Stand der Technik hinlänglich bekannt, beispielsweise das sog. Elektrospinnen.

Ebenso ist es möglich, zunächst einen Dorn vorzusehen, auf den eine 1. Membran aufgebracht wird. Anschließend wird die Gitterstruktur selbst auf die 1. Membran aufgebracht, bevor schließlich eine 2. Membran aufgetragen wird. Die einzelnen Streben, aus denen sich die Vorrichtung zusammensetzt, sind somit von Membranen eingeschlossen. In solchen Bereichen der Vorrichtung, in denen eine Membran nicht notwendig oder unerwünscht ist, kann diese anschließend noch entfernt werden.

Soweit von einer Membran die Rede ist, sei klargestellt, dass hierunter auch mehr als eine Membran verstanden werden kann. Es kann sich also um eine durchgehende Membran handeln, die größere Bereiche der Vorrichtung abdeckt, möglich ist jedoch auch der Einsatz mehrerer einzelner Membranen, die Bereiche der Vorrichtung abdecken. Diese einzelnen Membranen können aneinandergrenzen und so eine einheitliche Fläche ausbilden, es ist jedoch auch möglich, dass zwischen den Membranen Lücken verbleiben, solange das mit der Vorrichtung verbundene Ziel, ein Einfangen von Thrombusfragmenten zu erreichen, gewährleistet ist. Eine Membran kann mehrere Schichten aufweisen. Sofern mehrere Membranen oder Membranschichten übereinander angeordnet sind, ist auch dies insgesamt als Membran im Sinne der Erfindung zu verstehen.

Beim vorzugsweise angewendeten Elektrospinnen werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3.000 nm. Durch Elektrospinnen gewonnene Membranen sind sehr gleichmäßig ausgebildet. Die Membran ist zäh und mechanisch belastbar und kann mechanisch durchstoßen werden, ohne dass die Öffnung zum Ansatzpunkt für weitergehende Risse wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386 A1, die DE 28 06 030 A1 und die darin genannte Literatur hingewiesen. Statt durch Elektrospinnen kann die Membran auch über einen Tauch- oder Sprühprozess wie Spraycoaten hergestellt werden.

Die Membran kann aus einem Polymermaterial wie Polytetrafluorethylen, Polyester, Polyamiden, Polyurethanen oder Polyolefinen hergestellt sein. Besonders bevorzugt sind Polycarbonaturethane (PCU), die beispielsweise in einem Lösungsmittel wie Chloroform aufgebracht werden können. Wünschenswert ist insbesondere eine integrale Verbindung der Membran mit der Gitterstruktur. Die Haftung der Membran an den die Gitterstruktur bildenden Streben kann durch eine Silanisierung der Streben weiter verbessert werden.

Bei den Kopplungspunkten kann es sich um einfache Schweißpunkte handeln, an denen Einführhilfe/Führungsdraht und proximaler Abschnitt der Vorrichtung zusammengeführt sind. Denkbar sind auch übliche Kopplungselemente, die die Ablösung der Vorrichtung erlauben. Eine Ablösung ist zwar bei der Vorrichtung, anders als etwa bei Stents, normalerweise nicht vorgesehen, kann jedoch ausnahmsweise geboten sein, wenn eine Rückholung aus medizinischen Gründen nicht angezeigt ist, beispielsweise weil sie zu Schäden beim Patienten führen würde. In diesem Fall kann die Vorrichtung ähnlich einem Stent im Körper verbleiben und ihre Wirkung dadurch entfalten, dass sie im Thrombus einen Kanal ausbildet; der Thrombus wird durch die Maschenstruktur an die Gefäßwand gepresst, der Blutfluss bleibt aufgrund der Durchlässigkeit der Membran (mit oder ohne Öffnung) erhalten. Die Membran, die beispielsweise aus Polycarbonaturethan gefertigt sein kann, löst sich im Laufe der Zeit im Körper auf, sodass keine weitere Beeinflussung des Blutflusses erfolgt.

Ablösbare Kopplungselemente für Okklusionscoils oder Stents sind in der Literatur vielfältig beschrieben, insbesondere solche, die auf elektrolytischer, thermischer oder mechanischer Ablösung beruhen. Besonders bevorzugt sind elektrolytische Ablösesysteme, bei denen ein elektrolytisch korrodierbares Teil durch Stromeinwirkung aufgelöst wird und die Verbindung zwischen Vorrichtung und Einführhilfe durchtrennt. Geeignet als Ablöseelemente sind vorkorrodierte Edelstahlelemente, Magnesiumelemente oder Kobalt-Chrom-Legierungen. Bei einer mechanischen Ablösung besteht typischerweise ein Formschluss, der bei der Ablösung der Vorrichtung aufgehoben wird, so dass sich die Vorrichtung von der Einführeinheit löst.

Der Führungsdraht kann einstückig gefertigt sein, d. h. es handelt es sich letztlich um einen durchgehenden Draht. Möglich ist es aber auch, die vorteilhaften Eigenschaften unterschiedlicher Materialien miteinander zu kombinieren, bspw. den weiter proximal liegenden Teil des Führungsdrahts aus Edelstahl mit guter Vorschiebbarkeit, den weiter distal liegenden Teil des Führungsdrahts hingegen aus einer Nickel-Titan-Legierung wie Nitinol mit hoher Flexibilität zu fertigen. Ein distaler Abschnitt aus einer Nickel-Titan-Legierung weist auch den Vorteil auf, dass die Gefahr des Abknickens minimiert wird ("kink resistance"). Auf der anderen Seite ist für den proximalen Teil des Führungsdrahts die Verwendung eines steiferen Materials wie Edelstahl von Vorteil, weil dieses die Übertragung von Drehmomenten erlaubt, was für die Vorschiebbarkeit von Vorteil ist.

Der Begriff Einführhilfe ist weit zu verstehen und muss nicht in jedem Fall einen klassischen Führungsdraht bedeuten. Denkbar sind beispielsweise auch längliche Einführhilfen mit einem inneren Hohlraum.

Die Vorrichtung hat in der Regel eine Länge (ohne Einführhilfe) zwischen 5 mm und 40 mm und einen Durchmesser zwischen 1,5 mm und 7 mm, wobei sich die Dimensionen aus der Dimension des Blutgefäßes ergeben, aus dem der Thrombus entfernt werden soll. Die Angaben beziehen sich auf den freien, entspannten Zustand der Vorrichtung, d. h. ohne Ausübung eines äußeren Zwangs durch den Katheter. Die die Vorrichtung ausbildenden Streben können z. B. eine Breite bzw. einen Durchmesser zwischen 20 und 60 µm aufweisen.

Neben der Vorrichtung betrifft die Erfindung auch ein Verfahren zur Herstellung der Vorrichtung. Dieses ist dadurch gekennzeichnet, dass das Erzeugen der Einschnürung zwischen mittlerem und distalem Abschnitt bzw. die Herbeiführung einer Abrundung der Streben am distalen Ende des distalen Abschnitts in Form einer Wärmebehandlung erfolgt.

Die Erfindung wird anhand der in den Figuren veranschaulichten Ausführungsbeispiele beispielhaft näher erläutert. Es ist darauf hinzuweisen, dass die Figuren bevorzugte Ausführungsvarianten der Erfindung zeigen, die Erfindung ist jedoch nicht hierauf beschränkt. Allgemein umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: die Vorrichtung gemäß einer ersten Variante der ersten Ausführungsform in ausgerollter Form ohne Membran;
- Fig. 2 a: die Vorrichtung gemäß der ersten Variante der ersten Ausführungsform in der Seitenansicht ohne Membran;

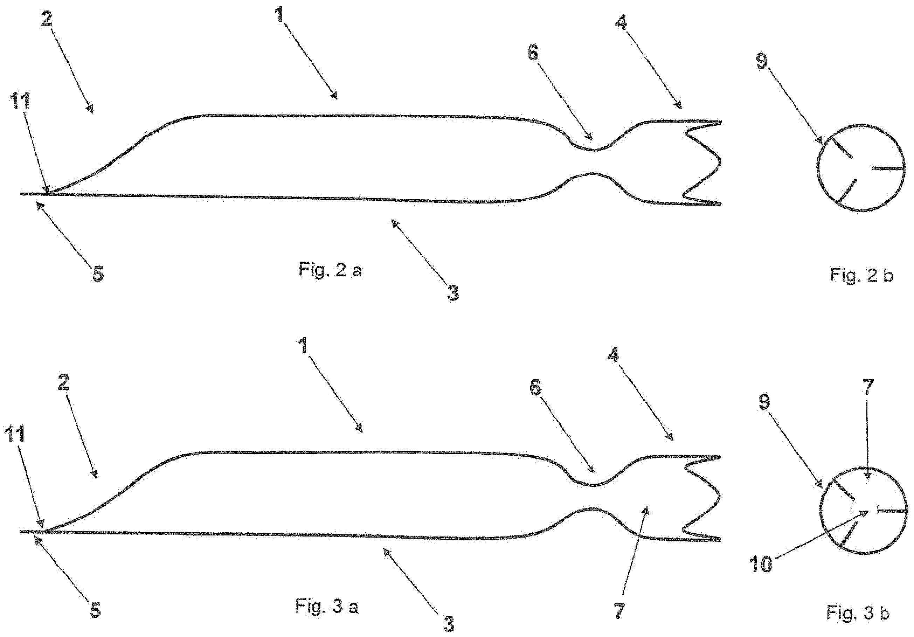

- Fig. 2 b: die Vorrichtung gemäß der ersten Variante der ersten Ausführungsform in der Frontalansicht ohne Membran;
- Fig. 3a: die Vorrichtung gemäß der ersten Variante der ersten Ausführungsform in der Seitenansicht mit Membran;
- Fig. 3b: die Vorrichtung gemäß der ersten Variante der ersten Ausführungsform in der Frontalansicht mit Membran;

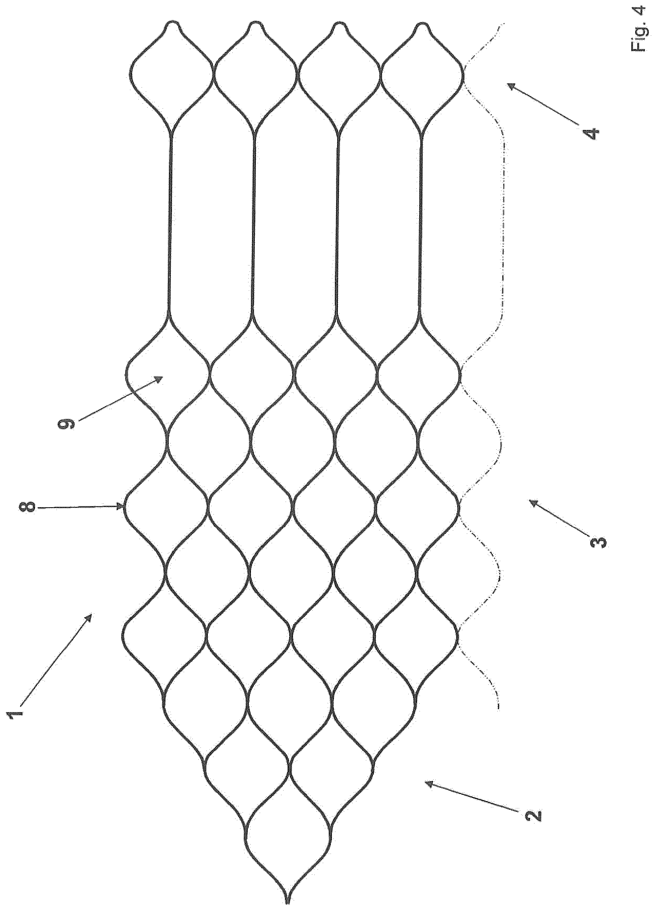

- Fig. 4: die Vorrichtung gemäß einer zweiten Variante der ersten Ausführungsform in ausgerollter Form ohne Membran;

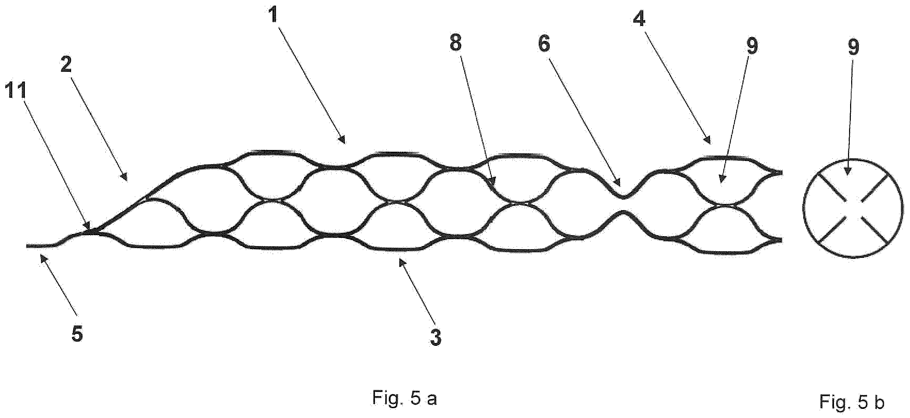

- Fig. 5 a: die Vorrichtung gemäß der zweiten Variante der ersten Ausführungsform in der Seitenansicht ohne Membran;
- Fig. 5 b: die Vorrichtung gemäß der zweiten Variante der ersten Ausführungsform in der Frontalansicht ohne Membran;

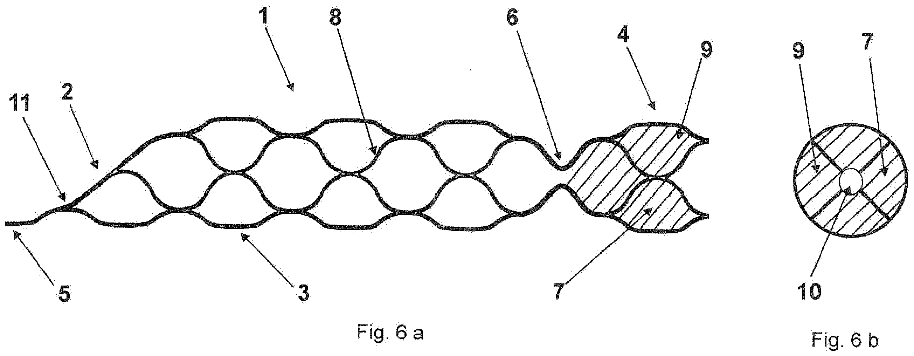

- Fig. 6 a: die Vorrichtung gemäß der zweiten Variante der ersten Ausführungsform in der Seitenansicht mit Membran gemäß der Erfindung;
- Fig. 6 b: die Vorrichtung gemäß der zweiten Variante der ersten Ausführungsform in der Frontalansicht mit Membran;

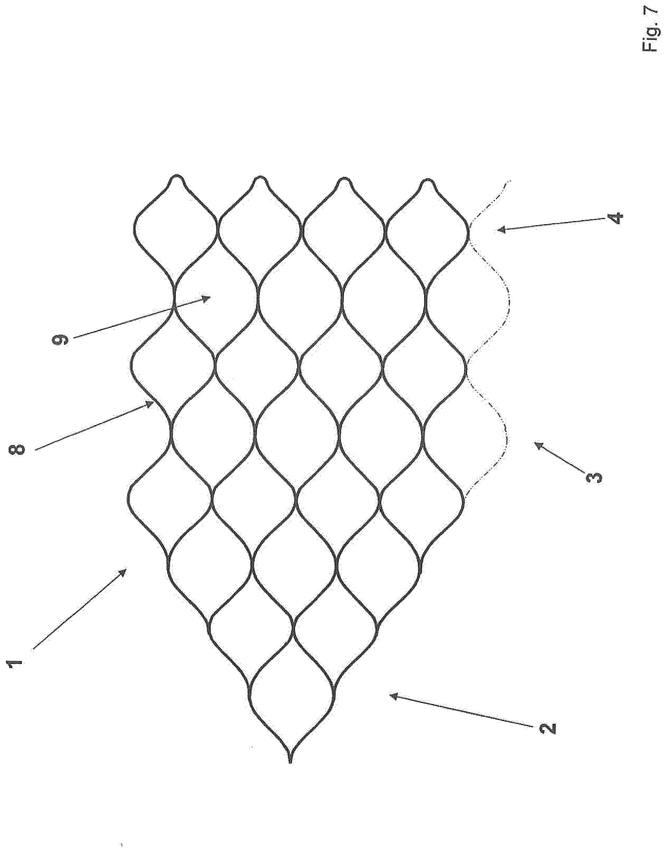

- Fig. 7: die Vorrichtung gemäß einer zweiten Ausführungsform in ausgerollter Form ohne Membran;

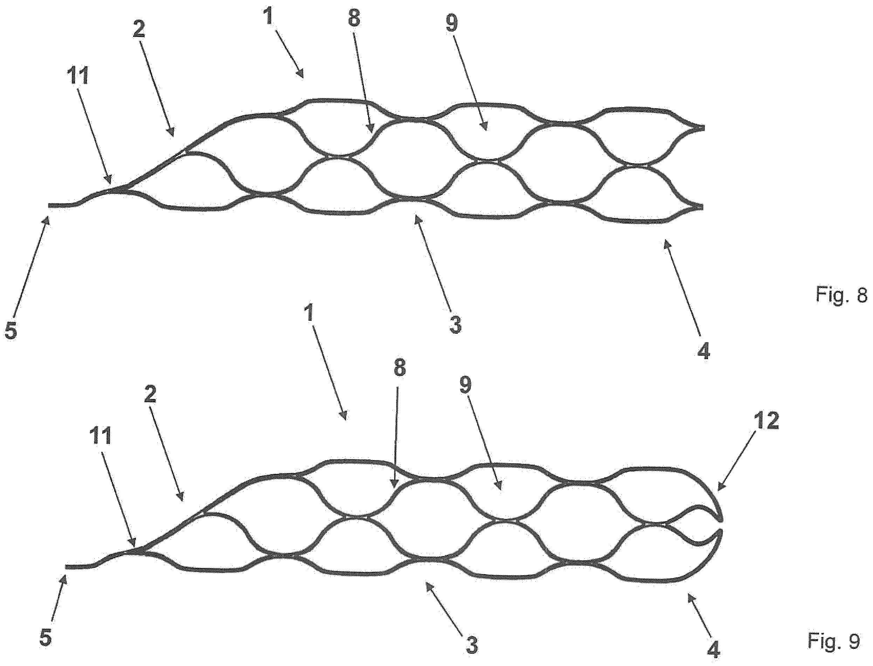

- Fig. 8: die Vorrichtung gemäß der zweiten Ausführungsform in der Seitenansicht ohne Membran vor Abrundung des distalen Endes;
- Fig. 9: die Vorrichtung gemäß der zweiten Ausführungsform in der Seitenansicht ohne Membran nach Abrundung des distalen Endes;

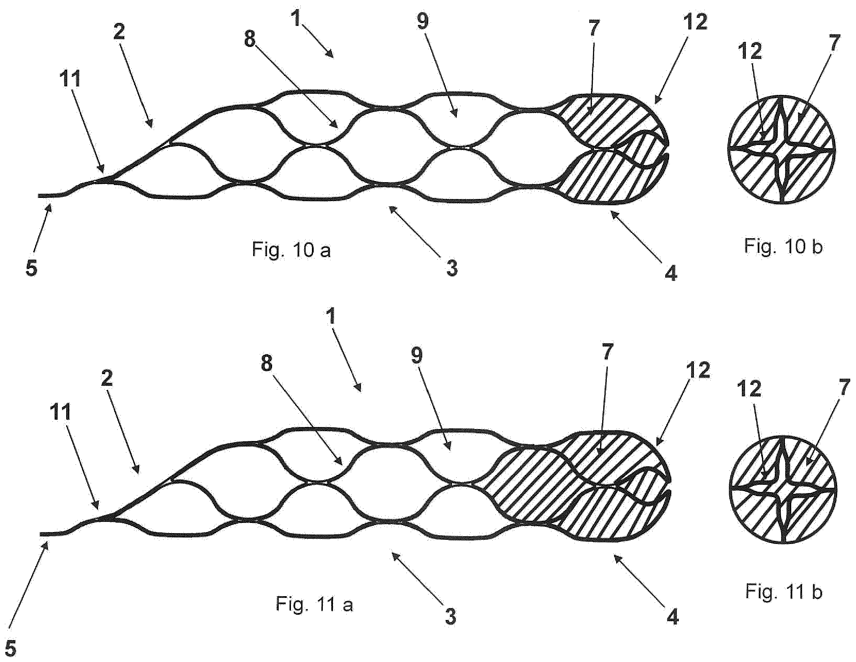

- Fig. 10 a: die Vorrichtung gemäß der zweiten Ausführungsform in der Seitenansicht mit Membran nach Abrundung des distalen Endes;
- Fig. 10 b: die Vorrichtung gemäß der zweiten Ausführungsform in der Frontalansicht mit Membran nach Abrundung des distalen Endes;
- Fig. 11 a: die Vorrichtung gemäß der zweiten Ausführungsform in der Seitenansicht mit Membran nach Abrundung des distalen Endes in einer Variante zu Fig. 10 a und
- Fig. 11 b: die Vorrichtung gemäß der zweiten Ausführungsform in der Frontalansicht mit Membran nach Abrundung des distalen Endes in einer Variante zu Fig. 10 b.

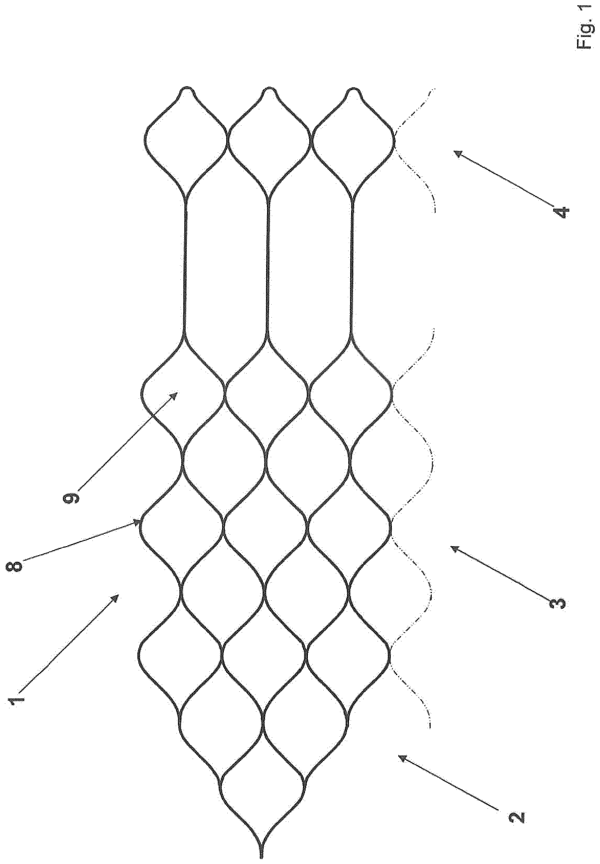

In Figur 1 wird die erfindungsgemäße Vorrichtung 1 gemäß einer ersten Variante der ersten Ausführungsform dargestellt, wobei die Vorrichtung 1 in ausgerollter Form noch ohne Membran vorliegt. Tatsächlich weist die Vorrichtung 1 selbstverständlich im Wesentlichen eine zylindrische Form auf. Die Vorrichtung 1 verfügt über einen proximalen, sich im Querschnitt verringernden und zusammenlaufenden Abschnitt 2, sowie über den mittleren, zylindrischen Abschnitt 3 und den distalen Abschnitt 4. Sie ist aus einzelnen Streben 8 aufgebaut, die offene Zellen 9 ausbilden. Die Zahl der orthogonal zur Längsachse der Vorrichtung 1 nebeneinander liegenden offenen Zellen 9 beträgt im vorliegenden Fall drei. Die angedeuteten strichpunktierten Linien deuten den Anschluss an die Streben 8 auf der gegenüberliegenden Seite der Vorrichtung 1 an.

Zwischen mittlerem Abschnitt 3 und distalem Abschnitt 4 befindet sich ein Bereich, in dem die Vorrichtung 1 weniger Streben 8 aufweist, der dafür vorgesehen ist, eine Einschnürung zu bilden, wenn die Vorrichtung 1 in eine zylindrische Form gebracht wird.

In Figur 2 a ist die Vorrichtung aus Figur 1 in der Seitenansicht gezeigt, jedoch noch ohne Membran. Darüber hinaus wurde auf die Darstellung der einzelnen offenen Zellen 9 verzichtet. Man erkennt wiederum den proximalen Abschnitt 2, den mittleren Abschnitt 3 und den distalen Abschnitt 4, wobei sich zwischen mittlerem Abschnitt 3 und distalem Abschnitt 4 die Einschnürung 6 befindet. Diese wird durch eine Wärmebehandlung der Streben 8 erzeugt. Am proximalen Ende läuft die Vorrichtung 1 zu einem Kopplungspunkt 11 aus, über den sie mit einer Einführhilfe 5 verbunden ist.

In Figur 2 b ist die Vorrichtung 1 aus den Figuren 1 und 2 a in einer Frontalansicht gezeigt, jedoch wiederum ohne die zur Erfindung gehörige Membran. Man erkennt, dass auf der Mantelfläche der Vorrichtung 1 jeweils drei offene Zellen 9 nebeneinander angeordnet sind.

Die Figur 3 a entspricht der Darstellung aus Figur 2 a, jedoch nachdem die Membran 7 auf die Vorrichtung 1 aufgebracht wurde. In der Frontalansicht in Figur 3 b erkennt man, dass die Membran 7 im Zentrum eine Öffnung 10 aufweist, welche dem Zweck dient, den Blutfluss dauerhaft aufrechtzuerhalten.

In Figur 4 ist eine Darstellung ähnlich der aus Figur 1 gewählt, um eine Variante der Vorrichtung 1 darzustellen. Grundsätzlich entspricht die Figur 4 der Figur 1, jedoch beträgt die Zahl der nebeneinander angeordneten offenen Zellen 9 in diesem Fall vier, wobei diese Zellen 9 selbstverständlich auf der Mantelfläche der Vorrichtung 1 radial nebeneinander liegen und insgesamt die zylindrische Struktur ausbilden.

In der Figur 5 a ist die Variante gemäß Figur 4 in der Seitenansicht, noch ohne Membran dargestellt. Anders als in der Figur 2 a werden hier auch die Streben 8 gezeigt, die die offenen Zellen 9 ausbilden. Man erkennt wiederum die Einschnürung 6 zwischen dem mittlerem Abschnitt 3 und dem distalen Abschnitt 4.

In der Figur 5 b ist die Vorrichtung aus Figur 5 a wiederum in der Frontalansicht gezeigt, ebenfalls ohne Membran. Man erkennt, dass die Zahl der offenen Zellen 9, die auf der Mantelfläche jeweils nebeneinander liegen, vier beträgt.

In Figur 6 a wird die Ausführungsform aus Figur 5 a gezeigt, diesmal jedoch nach Fertigstellung, d. h. mit Membran 7. In der entsprechenden Frontalansicht aus Figur 6 b erkennt man, dass die Membran 7 zentral über eine Öffnung 10 verfügt.

In der Figur 7 ist die Vorrichtung 1 gemäß der zweiten Ausführungsform noch ohne Membran dargestellt, bei der sich zwischen mittlerem Abschnitt 3 und distalem Abschnitt 4 keine Einschnürung befindet. Figur 7 stellt die Vorrichtung 1 in ausgerollter Form dar. Durch die strichpunktierten Linien wird angedeutet, dass sich in der Realität die gegenüberliegenden offenen Zellen 9 jeweils anschließen. Zum proximalen Ende hin läuft der proximale Abschnitt 2 eng zusammen.

Figur 8 zeigt den Entstehungsprozess der Vorrichtung gemäß der zweiten Ausführungsform aus Figur 7, wobei in Figur 8 die Vorrichtung 1 bereits in eine zylindrische Form gebracht wurde. Die die offenen Zellen 9 ausbildenden Streben 8 laufen am proximalen Abschnitt 2 letztlich in einem Kopplungspunkt 11 zusammen, über den die Vorrichtung 1 mit der Einführhilfe 5 verbunden ist. Die Erzeugung einer Abrundung des distalen Endes im distalen Abschnitt 4 ist in der Figur 8 noch nicht vollzogen worden.

Letzteres wird in Figur 9 dargestellt, d. h. hier sind die Streben 12 am distalen Ende des distalen Abschnitts 4 nach innen gebogen, so dass der distale Abschnitt 4 insgesamt eine Rundung oder Wölbung aufweist. Die Membran 7 wurde hier noch nicht angebracht.

In Figur 10 a schließlich ist die fertig gestellte Vorrichtung 1 aus den Figuren 7 bis 9 in der Seitenansicht dargestellt. Die Darstellung unterscheidet sich von der Darstellung aus Figur 9 dadurch, dass hier im distalen Abschnitt 4 die Membran aufgebracht wurde. Eine entsprechende Frontalansicht ist die Figur 10 b, in der man neben der Membran 7 auch die nach innen umgebogenen Streben 12 erkennt.

Figur 11 a ist eine Variante der Vorrichtung 1 aus Figur 10 a, bei der sich der distale Abschnitt 4, in dem offene Zellen 9 von einer Membran 7 bedeckt sind, weiter nach proximal erstreckt. Die Frontalansicht 11 b unterscheidet sich hingegen nicht von der zuvor gezeigten Variante aus Figur 10 b.

## Patentansprüche

1. Vorrichtung zur Entfernung von Thromben aus Blutgefäßen, wobei die Vorrichtung (1) in einem expandierten Zustand, in dem sie im Blutgefäß zeitweise freigesetzt wird, und in einem komprimierten Zustand vorliegt, in dem sie durch einen Mikrokatheter in das Blutgefäß eingeführt und aus diesem entfernt wird, wobei die Vorrichtung (1) über einen proximalen (2), einen mittleren (3) und einen distalen Abschnitt (4) verfügt, der mittlere Abschnitt (3) eine im Wesentlichen zylindrische Struktur aufweist und der proximale Abschnitt (2) an eine Einführhilfe (5) gekoppelt ist, wobei der distale Abschnitt (4) eine im Wesentlichen zylindrische Grundstruktur aufweist und zumindest teilweise über eine Abdeckung durch eine Membran (7) verfügt,
**dadurch gekennzeichnet, dass** sich zwischen mittlerem (3) und distalem Abschnitt (4) eine Einschnürung (6) befindet und die Membran (7) eine Trichterform aufweist, wobei das dünne Ende des Trichters zur Einschnürung (6) und das breite Ende in Richtung distal weist.

2. Vorrichtung nach Anspruch 1, **dadurch gekennzeichnet, dass** zumindest der mittlere Abschnitt (3) aus einer Vielzahl von über seine Mantelfläche verteilten offenen Zellen (9) aufgebaut ist.

3. Vorrichtung nach Anspruch 2, **dadurch gekennzeichnet, dass** die Zahl der offenen Zellen (9), die orthogonal zur Längsachse der Vorrichtung auf der Mantelfläche nebeneinander liegen, mindestens 2, vorzugsweise 3 bis 5 beträgt.

4. Vorrichtung nach einem der Ansprüche 1 bis 3, **dadurch gekennzeichnet, dass** die Membran (7) über zumindest eine Öffnung (10) verfügt.

5. Vorrichtung nach Anspruch 4, **dadurch gekennzeichnet, dass** die Öffnung (10) in der Membran (7) zentral angeordnet ist.

6. Vorrichtung nach einem der Ansprüche 1 bis 5, **dadurch gekennzeichnet, dass** die Einführhilfe (5) an einem oder mehreren Kopplungspunkten (11) an den proximalen Abschnitt (2) der Vorrichtung (1) gekoppelt ist, wobei der Kopplungspunkt (11) zur zentral verlaufenden Längsachse der Vorrichtung (1) radial in Richtung Mantelfläche verschoben angeordnet ist.

7. Vorrichtung nach einem der Ansprüche 1 bis 6, **gekennzeichnet durch** einen Schlitz, der sich in Längsrichtung der Vorrichtung (1) über die Mantelfläche zumindest eines Teils der Vorrichtung (1) erstreckt.

8. Vorrichtung nach Anspruch 7, **dadurch gekennzeichnet, dass** der Schlitz parallel zur Längsachse der Vorrichtung (1) verläuft.

9. Vorrichtung nach Anspruch 7, **dadurch gekennzeichnet, dass** der Schlitz wendelförmig über die Mantelfläche verläuft.

10. Vorrichtung nach einem der Ansprüche 1 bis 9, **dadurch gekennzeichnet, dass** zumindest einige Bereiche der Vorrichtung (1) mit einer röntgendichten Beschichtung versehen sind.

11. Verfahren zur Herstellung einer Vorrichtung (1) nach einem der Ansprüche 1 bis 10, **dadurch gekennzeichnet, dass** das Erzeugen einer Einschnürung (6) zwischen dem mittleren (3) und dem distalen Abschnitt (4) oder die Herbeiführung einer Abrundung der Streben (8) am distalen Ende des distalen Abschnitts (4) in Form einer Wärmebehandlung erfolgt.

## Claims

1. Device for removing thrombi from blood vessels, wherein the device (1) has an expanded state, in which it is temporarily released in the blood vessel and a compressed state, in which it is inserted into and removed from the blood vessel through a microcatheter, wherein the device (1) has a proximal portion (2), a central portion (3) and a distal portion (4); the central portion (3) is of substantially cylindrical structure and the proximal portion (2) is coupled to an insertion aid (5), wherein the distal portion (4) has a substantially cylindrical main structure and is at least partially provided with a covering in the form of a membrane (7),
**characterised in that**
a narrowed section (6) is situated between the central portion (3) and the distal portion (4), and the membrane (7) has a funnel shape, wherein the thin end of the funnel faces the narrowed section (6) and the wide end faces the distal direction.

2. Device according to claim 1, **characterised in that** at least the central portion (3) is made up of a plurality of open cells (9) distributed over its lateral surface.

3. Device according to claim 2, **characterised in that** the number of open cells (9) that are orthogonal to the longitudinal axis of the device and lie next to each other on the lateral surface is at least 2, preferably 3 to 5.

4. Device according to any one of claims 1 to 3, **characterised in that** the membrane (7) has at least one opening (10).

5. Device according to claim 4, **characterised in that** the opening (10) is arranged centrally in the membrane (7).

6. Device according to any one of claims 1 to 5, **characterised in that** the insertion aid (5) is coupled to the proximal portion (2) of the device (1) at one or more coupling points (11), wherein the coupling point (11) is arranged radially displaced in the direction of the circumferential surface with respect to the centrally extending longitudinal axis of the device (1).

7. Device according to any one of claims 1 to 6, **characterised by** a slit that extends over the lateral surface of at least part of the device (1) in the longitudinal direction of the device (1).

8. Device according to claim 7, **characterised in that** the slit runs parallel to the longitudinal axis of the device (1).

9. Device according to claim 7, **characterised in that** the slit runs helically over the lateral surface.

10. Device according to any one of claims 1 to 9, **characterised in that** some regions of the device (1) are provided with a radiopaque coating.

11. Method for producing a device (1) according to any one of claims 1 to 10, **characterised in that** the generation of a narrowed section (6) between the central portion (3) and the distal portion (4) or the rounding of the struts (8) at the distal end of the distal portion (4) is carried out by means of heat treatment.

## Revendications

1. Dispositif pour supprimer des thrombus de vaisseaux sanguins, dans lequel le dispositif (1) se trouve dans un état expansé, dans lequel il est libéré temporairement dans le vaisseau sanguin, et dans un état compressé, dans lequel il est introduit dans le vaisseau sanguin par un microcathéter et en est retiré, dans lequel le dispositif (1) dispose d'une section proximale (2), d'une section centrale (3) et d'une section distale (4), la section centrale (3) présente une structure sensiblement cylindrique et la section proximale (2) est couplée à une aide à l'insertion (5), dans lequel la section distale (4) présente une structure de base sensiblement cylindrique et dispose au moins en partie d'un recouvrement par une membrane (7),
**caractérisé en ce**
**qu'**un rétrécissement (6) se trouve entre la section centrale (3) et la section distale (4) et la membrane (7) présente une forme d'entonnoir, dans lequel l'extrémité fine de l'entonnoir pointe vers le rétrécissement (6) et l'extrémité large pointe dans la direction distale.

2. Dispositif selon la revendication 1, **caractérisé en ce qu'**au moins la section centrale (3) est élaborée à partir d'une pluralité de cellules ouvertes (9) réparties sur sa surface enveloppante.

3. Dispositif selon la revendication 2, **caractérisé en ce que** le nombre des cellules ouvertes (9), qui sont côte à côte sur la surface enveloppante de manière orthogonale par rapport à l'axe longitudinal du dispositif, est d'au moins 2, de préférence va de 3 à 5.

4. Dispositif selon l'une quelconque des revendications 1 à 3, **caractérisé en ce que** la membrane (7) dispose d'au moins une ouverture (10).

5. Dispositif selon la revendication 4, **caractérisé en ce que** l'ouverture (10) est disposée de manière centrale dans la membrane (7).

6. Dispositif selon l'une quelconque des revendications 1 à 5, **caractérisé en ce que** l'aide à l'insertion (5) est couplée à un ou à plusieurs points de couplage (11) sur la section proximale (2) du dispositif (1), dans lequel le point de couplage (11) est disposé de manière décalée radialement en direction de la surface enveloppante par rapport à l'axe longitudinal s'étendant de manière centrale du dispositif (1).

7. Dispositif selon l'une quelconque des revendications 1 à 6, **caractérisé par** une entaille qui s'étend sur la surface enveloppante d'au moins une partie du dispositif (1) dans le sens longitudinal du dispositif (1).

8. Dispositif selon la revendication 7, **caractérisé en ce que** l'entaille s'étend de manière parallèle par rapport à l'axe longitudinal du dispositif (1).

9. Dispositif selon la revendication 7, **caractérisé en ce que** l'entaille s'étend en forme de spirale sur la surface enveloppante.

10. Dispositif selon l'une quelconque des revendications 1 à 9, **caractérisé en ce qu'**au moins certaines zones du dispositif (1) sont pourvues d'un revêtement étanche aux rayons X.

11. Procédé pour fabriquer un dispositif (1) selon l'une quelconque des revendications 1 à 10, **caractérisé en ce que** la production d'un rétrécissement (6) a lieu entre la section centrale (3) et la section distale (4) ou l'obtention d'un arrondi des entretoises (8) sur l'extrémité distale de la section distale (4) a lieu sous la forme d'un traitement thermique.
